# EUROPEAN PATENT APPLICATION

(11) **EP 4 725 479 A1**
(43) Date of publication of application: **15.04.2026**
(21) Application number: 24205578.8
(22) Date of filing: 09.10.2024
(51) Int. Cl.: A61K 31/192, A61K 31/216, A61K 31/4184, A61P 1/16, A61P 11/00

(54) **PPAR AGONISTS FOR TREATMENT OF DISEASES ASSOCIATED WITH AATD**

(71) Applicant: Max-Planck-Gesellschaft zur Förderung der Wissenschaften e.V., 80539 München (DE)
(72) Inventor: The designation of the inventor has not yet been filed
(74) Representative: Weickmann & Weickmann PartmbB

(57) **Abstract**

The present invention relates to a pharmaceutical composition comprising an agent for enhancing peroxisomes for use for treatment of a condition associated with alpha-1-antitrypsin deficiency (AATD).

## Description

The present invention relates to a pharmaceutical composition comprising an agent for enhancing peroxisomes for use for prevention and/or treatment of a condition associated with and/or caused by alpha-1-antitrypsin deficiency (AATD).

Alpha-1-antitrypsin deficiency (AATD) is a genetic disorder effecting protein misfolding, resulting in diseases of liver and lung. AATD is caused by autosomal co-dominant mutations in the SERPINA1 gene, leading to misfolding and accumulation of alpha-1 antitrypsin (AAT) in hepatocytes, the primary liver cells responsible for AAT production. The most severe cases of AATD are associated with a homozygous Z-variant (Pi*ZZ genotype), with a peak incidence of 1:2,000 in Caucasian populations (Greene, C.M. et al. (2016), Nature Reviews. Disease Primers, 2, p. 16051; Strnad, P. et al. (2022), New England Journal of Medicine, 387(6), pp. 514-524).

AATD primarily affects two major organ systems: the lungs and the liver. Lung complications, including emphysema, are linked to a systemic decrease of AAT in blood plasma due to defective secretion from hepatocytes. In the liver, AAT misfolding and accumulation in hepatocytes can lead to cell death and trigger liver fibrosis. Despite sharing the same underlying genetic mutation, patients with AATD exhibit significant variability in liver disease progression and severity. Approximately 35% of homozygous Pi*ZZ patients develop severe liver fibrosis up to liver cirrhosis, potentially requiring liver transplantation, while many others do not develop liver symptoms. The reasons for this clinical heterogeneity are unclear, and understanding these factors can guide the development of targeted therapeutic interventions.

A number of advanced treatments for AATD have entered clinical trials. For instance, the RNA interference therapeutic Fazirsiran (Arrowhead Pharmaceuticals and Takeda) was successfully tested in a phase 2 clinical trial (Strnad, P., McElvaney, N.G. and Lomas, D.A. (2020), New England Journal of Medicine, 382(15), pp. 1443-1455) and has recently entered a phase 3 trial (https://clinicaltrials.qov/study/NCT05677971). The RNA editing oligonucleotide WVE-006 (Wave Life Sciences) has shown good efficacy in a stem cell and mouse model, and is currently tested in a phase 1b/2a open label study. While this exemplary progress can pave the way towards decreasing the disease burden of AATD, the problem of clinical heterogeneity will not be solved and each class of drugs encompasses clinical problems. For instance, RNA interference drugs lower the amount of systemic AAT, which can have adverse side-effects in the lung. Modifying oligonucleotides only enter some cells, still rendering other cells untreated. A further or alternative medication can thus be of great clinical benefit in precisely adjusting disease activity in AATD patients.

The identification of druggable pathways has been hampered by the lack of appropriate model system. While murine models and patient-derived induced pluripotent stem cells (iPSCs) are frequently used in AATD-related research, they do not fully recapitulate the disease heterogeneity in the human population (Yusa et al., (2011), Nature, 478 (7369), pp 391-394).

It was therefore an object of the present invention to identify factors influencing development or prevention of conditions or diseases associated with AATD, such as severe liver fibrosis in patients having AATD, and in particular to provide active agents for prevention and/or treatment of conditions associated with and/or caused by AATD.

According to the invention it has been found that upregulation of peroxisomes is correlated with patients having AATD but facing no or only low-grade liver fibrosis.

The present invention therefore relates to a pharmaceutical composition comprising an agent for enhancing peroxisomes for use for prevention and/or treatment of a condition or disease associated with and/or caused by alpha-1-antitrypsin deficiency (AATD).

The finding underlying the present invention starts from the known but still unresolved fact that patients having AATD show symptoms, in particular liver fibrosis and/or liver cirrhosis, to quite a different severity degree as well as with varying disease onset. Due to this special characteristic of AATD, animal models and also trials in patients do not give satisfying results reflecting the disease heterogeneity. According to the present invention, therefore, spatial proteomics by mass spectrometry was employed. A patient collective, all having AATD but with varying degree of symptoms, was examined. The patient collective was divided into several groups according to the severity of liver fibrosis. Using spatial proteomics by mass spectrometry, proteins reacting to accumulation of AAT were identified and reactions were stratified by patients' fibrosis stage. As a surprising result, a strong peroxisome response was identified in the group showing low symptoms. Thus, the invention resides in the finding that enhancement of peroxisomes is the key in preventing development of severe symptoms in patients having AATD. According to the present invention, an agent for enhancing peroxisomes is provided for use for prevention and/or treatment of a condition associated with and/or caused by AATD.

In particular, it was found according to the present invention that using spatial proteomics by mass spectroscopy molecular events in patients having AATD but showing symptoms thereof, in particular liver disease progression and severity, to varying degrees, can be observed, measured and assigned. Herein, a pseudo-time resolved proteome of individual hepatocytes undergoing proteotoxic stress due to AAT aggregation is provided.

Using spatial proteomics by mass spectrometry, molecular events in intact patient tissue using formalin-fixed and paraffin-embedded (FFPE) samples were observed. The findings derived from FFPE biopsies and resections from patients provide novel insights into the progression and hepatic manifestation in AAT deficiency. The responses to AAT accumulation across the full disease spectrum (fibrosis stage F1 - F4) in human patient samples were captured. Thereby it was found that peroxisomal proliferation is a protective hallmark of hepatocytes in alpha-1 antitrypsin deficiency. The methods applied, in particular single-cell spatial proteomics by mass spectrometry using formalin-fixed and paraffin-embedded (FFPE) samples (single-cell Deep Visual Proteomics (scDVP) of formalin-fixed tissue), enable characterization of the entire proteomic and phenotypic life cycle of stressed hepatocytes in a proteotoxic and fibrogenic liver disease. By this method, known disease progression markers could be recapitulated, while additional dysregulated proteins were identified. In particular, an early upregulation of the peroxisome compartment in samples from patients having low-grade liver fibrosis was surprisingly found. This response is significantly delayed in high-grade fibrotic samples, opening a window for therapeutic intervention.

Alpha-1 antitrypsin deficiency (AATD) is a genetic disorder causing variable clinical outcomes, from no/mild liver fibrosis to liver cirrhosis requiring transplantation. The reasons for this heterogeneity are unclear. According to the invention it was now found that upregulation of peroxisomes is an early event during AAT accumulation specific to patients with low-grade fibrosis that is much reduced or absent in patients that have developed sever liver fibrosis. This molecular response is e.g. inducible with PPARα agonists, in particular with fibrates.

The data obtained show that peroxisomal activation is an early response to cell stress due to AAT accumulation in hepatocytes, which has not been described before in the case of AATD. Furthermore, the signature is characteristic for low-grade fibrosis patient samples and can therefore explain the clinical heterogeneity observed. Further, prominent elevation of the protein ERO1A was found. These are part of the unfolded protein response and produce hydrogen peroxide (H₂O₂) as a membrane-permeable byproduct, that can be detoxified by the peroxisomal protein catalase.

In particular, the present invention relates to a pharmaceutical composition comprising an agent for enhancing peroxisomes for use for prevention and/or treatment of a condition associated with and/or caused by alpha-1-antitrypsin deficiency (AATD), wherein the agent for enhancing peroxisome is a peroxisome proliferator-activated receptor (PPAR) agonist.

### Agent for enhancing peroxisomes

Peroxisomes are membrane-bound organelles which are found in the cytoplasm of eukaryotic cells. Peroxisomes are oxidative organelles which show hydrogen peroxide generating and scavenging activities.

An agent for enhancing peroxisomes in the sense of the present invention is any agent or compound which enhances or increases the number and/or functionality of peroxisomes. A preferred agent for enhancing peroxisomes according to the present invention is a peroxisome proliferator-activated receptor agonist (PPAR agonist).

Peroxisome proliferator-activated receptors (PPARs) are a group of nuclear receptor proteins that function as transcription factors increasing peroxisomal load. PPAR agonists are agents or compounds which act upon the peroxisome proliferator-activated receptor. There are three main types of PPARs: PPAR-α, PPAR-γ, and PPAR-δ (also known as PPAR-β/δ).

Preferably according to the present invention, the agent for enhancing peroxisomes is a peroxisome proliferator-activated receptor (PPAR) agonist selected from the group of PPAR-α agonists, PPAR-γ agonists, PPAR-δ agonists and/or PPAR-pan agonists. Most preferred, the agent for enhancing peroxisomes is a PPAR-α agonist.

Drugs that activate these receptors are used to treat a variety of metabolic and inflammatory diseases, including hypertriglyceridemia and type 2 diabetes mellitus. Particularly the PPAR-α agonists, exemplified by fibrates, increase the load of peroxisomes, PEX proteins and catalase in the liver. This class of drugs has well-established safety profiles, making it suitable for treating patients with late-diagnosed advanced liver fibrosis due to AATD.

PPAR-δ mobilates plasma lipid levels through regulation of fatty acid oxidation and also regulates glucose handling.

PPAR-δ agonists useful in the present invention in particular include GW0742; GW501516; KD3010; L165041; Seladelpar; Telmisartan; and/or 12 ((E)-2-(4-((3-(4-fluorophenyl)-3-(4-(3-morpholinoprop-1-yn-1-yl)phenyl)allyl)oxy)-2-methyl phenoxy) acetic acid).

PPAR pan/dual agonists useful in the present invention include Aleglitazar; Bezafibrate; Chiglitazar; Elafibranor; Lanifibranor; Lobeglitazone; Muraglitazar; Naveglitazar; Saroglitazar; Tesaglitazar; and/or ZLY18.

PPAR-α is a nuclear receptor activated by peroxisome proliferators. PPAR-α is specifically active in the liver. PPAR-α agonists useful in the present invention in particular include 7(S)-hydroxydocosahexaenoic acid, elifibranor, and in particular fibrates. Preferred PPAR-α agonists include Bezafibrate; Clofibrate; Ciprofibrate; Fenofibrate; Fenofibric acid; Gemfibrozil; Oleoylethanolamide; Pemafibrate; 3-Hydroxy-2,2-dimethylbutyrate; 9-Hydroxy-10(E),12(E)-octadecadienoic acid; 9-Octadecenamide; Hexadecanamide; GW7647; LY-674; LY518674; and/or Wy-14643.

With the advent of siRNA therapeutics and modifying oligonucleotides, strong candidates for treatment of AATD are already on the way. These drugs will be, however, very expensive, and a cost-efficient treatment for short- to mid-term AATD management can significantly lower the burden on national health care systems, especially in countries with limited society-funded health care. Therefore, according to the present invention, peroxisomal activators are provided for use for treatment of alpha-1 antitrypsin deficiency.

### AATD

According to the present invention an atypical increase in the peroxisomal compartment was observed for patients having AATD but only low-grade fibrosis. Accordingly, conditions associated with alpha-1-antitrypsin deficiency (AATD) can be treated by an agent for enhancing peroxisomes. Alpha-1 antitrypsin deficiency (AATD) is a protein misfolding disease which poses significant health challenges, with its cellular progression still poorly understood. The disease alpha-1 antitrypsin deficiency (AATD), is a genetic disorder caused by autosomal, co-dominant mutations in the *SERPINA1* gene resulting in misfolding and accumulation of alpha-1 antitrypsin (AAT) in hepatocytes, related to liver fibrosis. Most severe AATD cases are caused by a homozygous Z-variant (Pi*ZZ genotype) with a peak incidence of 1:2,000 in individuals of European descent. Current hypotheses suggest that the severity of liver damage correlates with the amount of accumulated AAT. However, the mechanisms driving fibrogenesis or hepatocyte survival versus death remain unclear, leaving potentially druggable targets unexplored. Conditions associated with alpha-1-antitrypsin deficiency (AATD) are in particular liver diseases and/or lung diseases.

According to the present invention, a condition associated with and/or caused by alpha-1-antitrypsin deficiency (AATD) can be prevented and/or treated. In particular, a condition or disease for which AATD is a primary cause can be prevented and/or treated.

Liver diseases associated with AATD which can be prevented and/or treated according to the present invention include in particular proteotoxic and/or fibrogenic liver diseases associated with AATD. Most preferably, the present invention relates to a pharmaceutical composition comprising an agent for enhancing peroxisome for use for prevention and/or treatment of liver fibrosis and/or liver cirrhosis caused by AAT proteotoxicity in AATD.

For the conditions to be treated AATD is the primary cause while the symptoms seen, in particular a liver disease such as liver fibrosis, is secondary. According to the present invention, an agent for enhancing peroxisomes and in particular PPAR agonists primarily helps the cell to cope these peroxides, thus avoiding buildup of fibrotic material.

As found according to the present invention, hepatocytes in patients having AATD are intrinsically fibrogenic. This means that misfolded AAT accumulates in hepatocytes resulting in that the hepatocytes themselves are fibrogenic. This intrinsic hepatocyte-specific response causes formation of extracellular fibrotic scars resulting in liver fibrosis. This mechanism can be prevented and/or countered by an agent for enhancing peroxisomes, in particular a PPAR agonist and more preferably a PPAR alpha agonist. The enhanced peroxisomal response in hepatocytes is a protective factor. It was found that a higher peroxisomal activity in hepatocytes is correlated with reduced liver fibrosis in patients having AATD. Without being bound to a theory, it is assumed that the mechanism underlying the effectiveness of an agent for enhancing peroxisomes in the prevention and/or treatment of a condition associated with and in particular caused by AATD is that cells attempt to rescue misfolded AAT. However, the cellular response causes stress by formation of peroxides. These peroxides are scavenged when enhancing peroxisomes, thus, the peroxisomes act as self-intrinsic protective factor.

Thus, according to the present invention, formation of liver fibrosis can be prevented and the agent for enhancing peroxisomes counteracts hepatocyte-derived fibrinogenic activities. The agent for enhancing peroxisomes and in particular a PPAR agonist functions to prevent and/or treat a fibrinogenic action, which is intrinsic to the hepatocytes of subjects having AATD.

This is in contrast to clinical conditions associated with fibrotic liver diseases, such as alcohol-associated liver disease (ALD), Metabolic dysfunction-associated steatohepatitis (MASH), Metabolic-dysfunction associated fatty liver disease (MALFD) or virus-induced liver fibrosis, in which extrinsic factors cause cellular stress, invasion of immune cells and inflammation into liver tissue, resulting in deposition of connective tissue and organ hardening.

As a result, the agent for enhancing peroxisomes, in particular a PPAR agonist and preferably a PPAR alpha agonist can be used in the prevention and/or treatment, in particular for prevention, of a condition or disease associated with and/or caused by alpha-1 antitrypsin deficiency (AATD). The condition is in particular caused by alpha-1 antitrypsin deficiency (AATD), wherein AATD is in particular a primary cause for the condition or the disease.

The agent for enhancing peroxisomes, in particular a PPAR agonist, is preferably comprised in the pharmaceutical composition of the present invention in an amount of from 10mg to 1000mg, preferably from 20 to 800 mg, or preferably from 10mg to 1000mg retard or preferably from 10mg to 500mg non-retard.

The agent for enhancing peroxisome is administered according to the present invention preferably in a daily amount of from 10mg up to 1600mg, in particular from 10mg up to 1000mg up to twice daily.

### Pharmaceutical compositions

The pharmaceutical composition according to the present invention preferably comprises an agent for enhancing peroxisomes, in particular a PPAR agonist, optionally together with a pharmaceutically acceptable carrier, vehicle, excipient and/or diluent.

"Pharmaceutical composition" refers to a preparation in a form that allows the biological activity of the active ingredient(s) to be effective, and which contains no additional components which are toxic to the patients to which the formulation is administered. As used herein, the term "pharmaceutically acceptable" means that the materials are suitable for administration to or upon a mammal, in particular a human, without production of undesirable physiological effects. "Pharmaceutically acceptable carrier" in particular refers to an ingredient in a pharmaceutical composition, other than an active ingredient, which is nontoxic to the subject to whom it is administered. A pharmaceutically acceptable carrier includes, but is not limited to, a buffer, excipient, stabilizer, or preservative.

The agent for enhancing peroxisomes and the pharmaceutical composition comprising such an agent for enhancing peroxisomes, respectively, are preferably administered orally, topically, parenterally, inhaled, intranasally or i.v.

For oral application the pharmaceutical composition can be formulated into powders, granules, tablets, capsules, suspensions, emulsions, syrups or any other oral dosage form. It can be prepared using a diluent or excipient such as generally used fillers, extenders, binders, wetting agents, disintegrating agents, surface active agents. A solid preparation for oral administration may be a tablet, pill, powder, granule, or capsule. The solid preparation may further comprise an excipient. Excipients may be, for example, starch, calcium carbonate, sucrose, lactose, or gelatine. In addition, the solid preparation may further comprise a lubricant, such as magnesium stearate, or talc.

For preparing solid compositions such as tablets, the agent for enhancing peroxisomes can be mixed with a pharmaceutically acceptable carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid pre-formulation composition containing a homogeneous mixture. In such a homogeneous pre-formulation composition the agent for enhancing peroxisomes is dispersed evenly throughout the composition so that the composition may be easily subdivided into equally effective unit dosage forms such as tablets, pills and capsules. This solid pre-formulation composition is then subdivided into unit dosage forms containing from 0.1 mg to about 500 mg of the active ingredient. Typical unit dosage forms contain from 1 mg to 1000 mg, in particular from 2 mg, preferably from 5 mg and up to 500 mg, preferably up to 200 mg und more preferably up to 100 mg of agent for enhancing peroxisomes.

Liquid preparations for oral administration may be suspensions, solutions, emulsions, or syrups. The liquid formulation may comprise water, or liquid paraffin. The liquid formulation may, for example, include excipients, such as wetting agents, sweeteners, aromatics or preservatives. The liquid forms in which the composition of the present invention may be incorporated for administration orally include aqueous solutions, suitably flavored syrups, aqueous or oil suspensions, and flavored emulsions with edible oils such as cottonseed oil, sesame oil, coconut oil or peanut oil, as 15 well as elixirs and similar pharmaceutical vehicles. Suitable dispersing or suspending agents for aqueous suspensions include synthetic and natural gums such as tragacanth, acacia, alginate, dextran, sodium carboxymethylcellulose, Methylcellulose, polyvinylpyrrolidone or gelatin.

In a further preferred embodiment, the agent for enhancing peroxisomes and the pharmaceutical composition comprising an agent for enhancing peroxisomes, respectively, are preferably administered topically, in particular cutaneous-topically. Such formulations in particular allow the active agent to penetrate the skin. For topical and in particular for cutaneous-topical application, the pharmaceutical composition can be formulated as liquid, semi-solid or solid preparation which is applied to the skin in the form of a creme, solution, emulsion, suspension, paste, ointment, gel, foam or powder, preferably in form of a creme or ointment.

Suitable carriers for topical, in particular for cutaneous-topical administration include propylene glycol, C13-C20 alcohols, in particular cetyl alcohol or stearyl alcohol, paraffine, triglycerides and hydroxy benzoates, in particular methyl-4-hydroxy benzoate or propyl-4-hydroxy benzoate.

The agent for enhancing peroxisomes may also be administered parenterally, in particular into the blood stream, into muscle or into an internal organ. For parenteral administration, preferably an aqueous solution is provided which may contain pharmaceutically acceptable excipients.

For intranasal administration or for administration by inhalation, the pharmaceutical composition is preferably provided in the form of a dry powder.

The present invention further relates to an agent for enhancing peroxisomes, in particular a peroxisome proliferator-activated receptor (PPAR) agonist, for use for treatment of a disease associated with alpha-1-antitrypsin deficiency (AATD).

The present invention also relates to the use of an agent for enhancing peroxisomes, in particular of a peroxisome proliferator-activated receptor (PPAR) agonist, for the manufacture of a medicament for treating a disease associated with alpha-1-antitrypsin deficiency (AATD).

The present invention further relates to a method for treatment of a disease associated with alpha-1-antitrypsin deficiency (AATD) comprising administering an agent for enhancing peroxisomes, in particular a peroxisome proliferator-activated receptor (PPAR) agonist.

The appended figures and the following example further illustrate the invention.

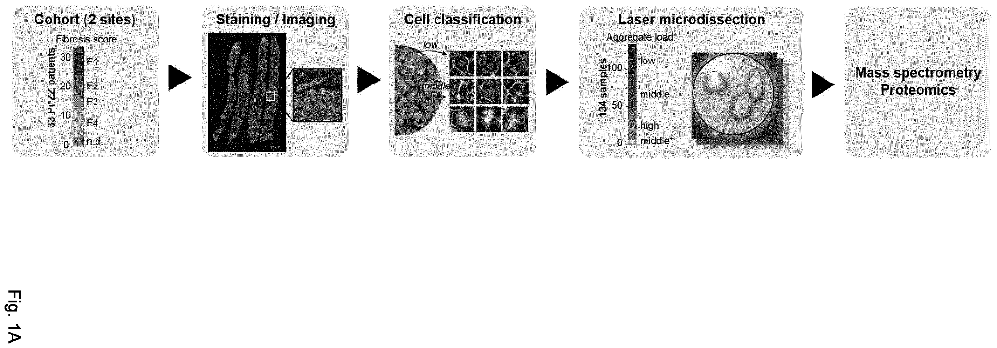

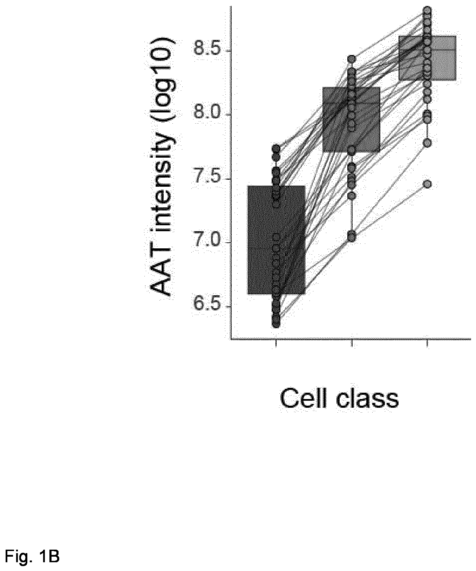

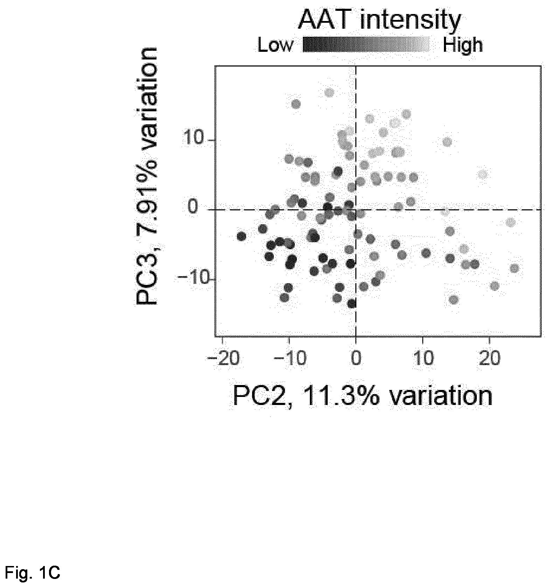

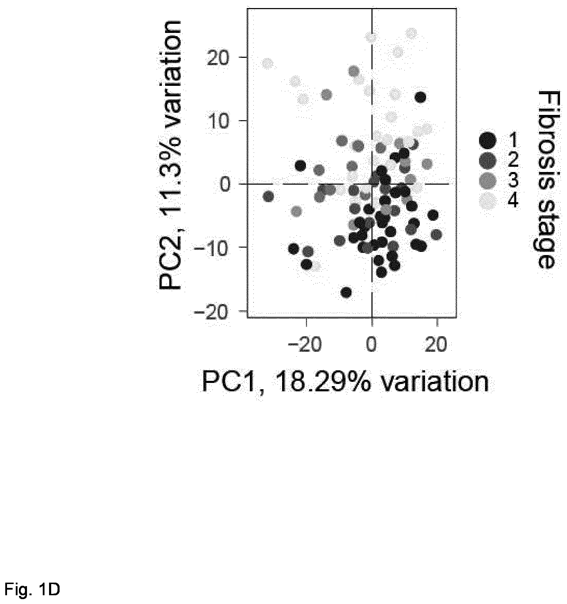

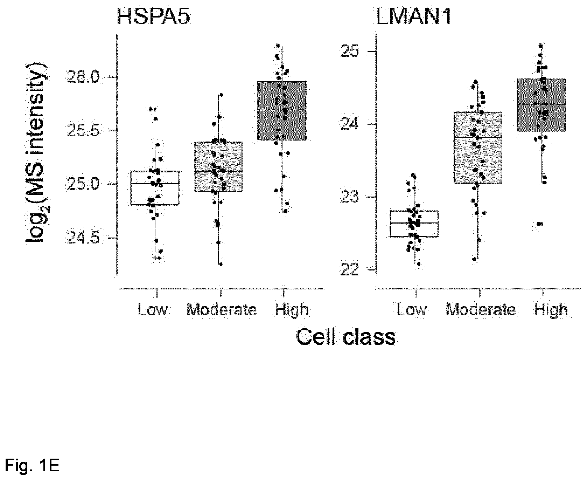

Figure 1. Proteomic mapping of hepatocyte stress response. A, Overview of the Deep Visual Proteomics workflow. Fibrosis stages are Kleiner scores. B, MS intensity of AAT across three classes. One dot is one sample from a patient (n = 32). C, Principal component analysis with principal components 2 and 3 colored by AAT levels, and D, with principal component 1 and 2 colored by fibrosis stage. Each dot is one sample (n = 95). E, MS intensity of selected proteins across three classes. One dot is one sample from a patient (n = 32).

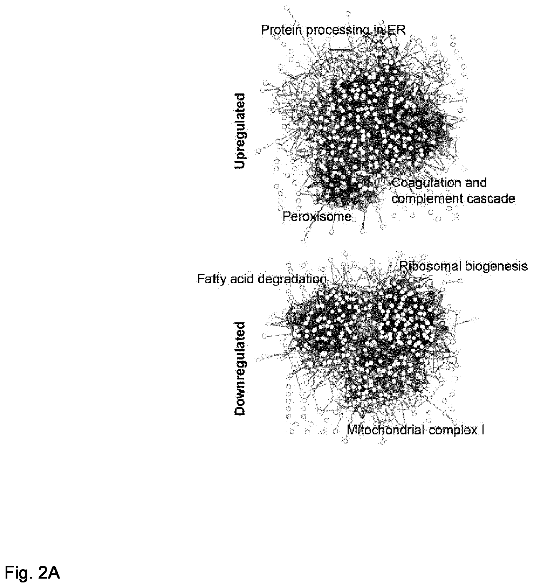

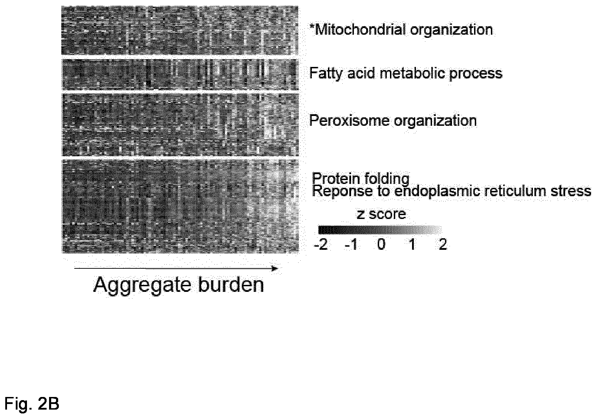

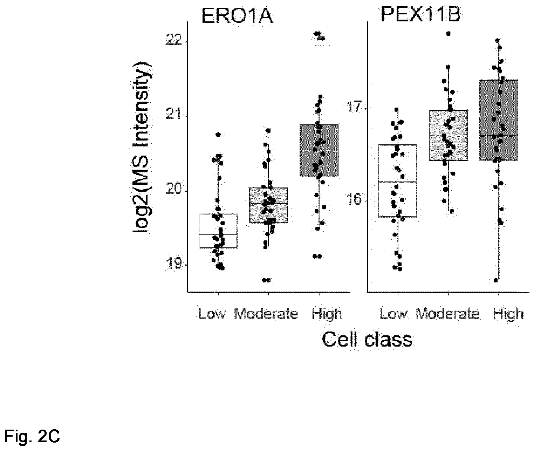

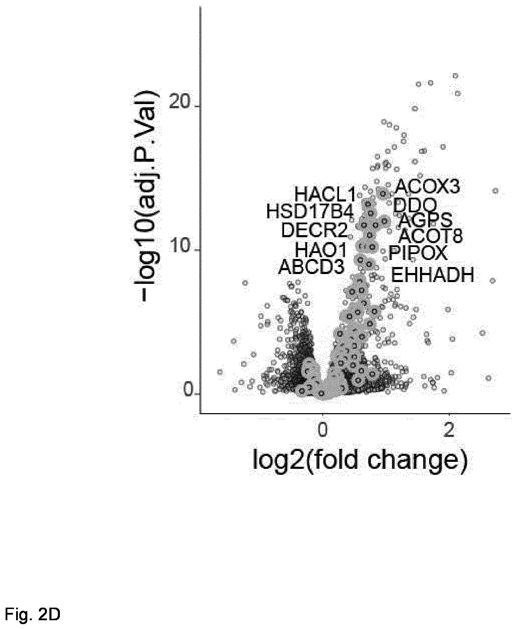

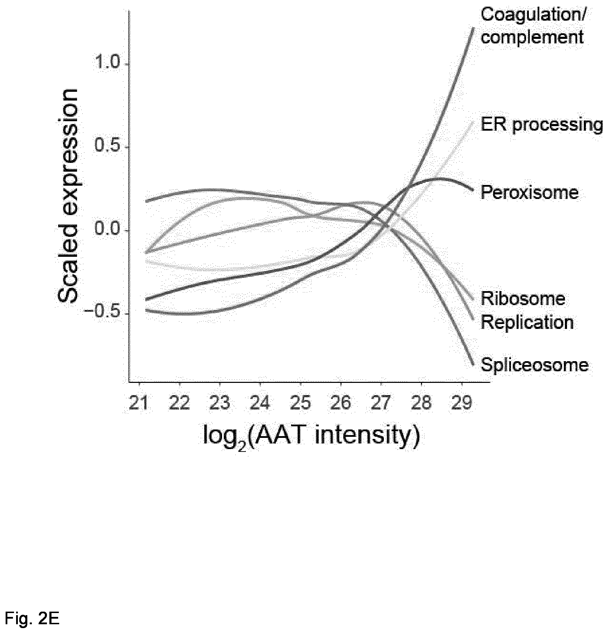

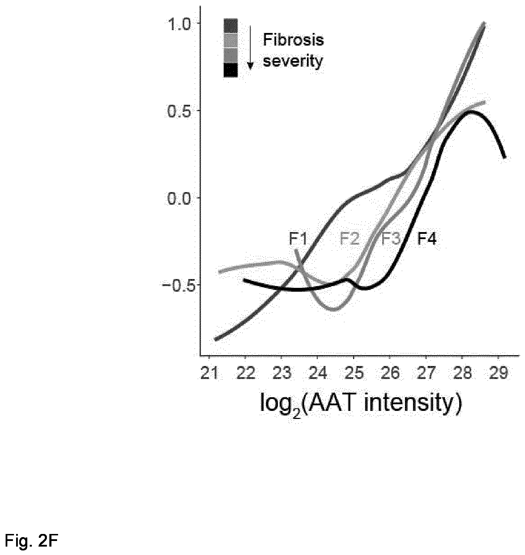

Figure 2. Peroxisomal proliferation is a protective hallmark of hepatocytes in alpha-1 antitrypsin deficiency. A, STRING interaction network of significantly (FDR < 0.05) upregulated (top) or downregulated proteins in cells with AAT aggregates. B, Clustering into early and late responding genes to proteotoxic stress, order on x axis by AAT levels. The y axis was broken into groups to achieve good coge of all response types. Significant KEGG term per box are shown, *not significant. C, MS intensity of selected protns across three classes. One dot is one sample from a patient (n = 32). D, Statistics of protein expression upon aggregate formation. Highlighted in orange are peroxisomal proteins. E, Summarised levels of peroxisomal and proteins in other functional categories expressed as z-scores across all samples (n = 32), with AAT levels on the x axis. F, Summarised levels of peroxisomal proteins expressed as z-scores across all samples (n = 32), split by fibrosis stage and with AAT levels on the x axis. F1: low fibrosis stage, F4: high fibrosis stage.

### EXAMPLES

### Example 1

To elucidate the molecular basis of the observed clinical heterogeneity in AATD patients, a comprehensive proteomic mapping approach was employed to characterize hepatocyte responses. We assembled a cohort of 32 AATD patient samples from two clinical sites (Odense and Aachen) who provided formalin-fixed and paraffin-embedded sections or blocks. After staining for cell outlines and AAT, hepatocytes were segmented and stratified into low, moderate, and high aggregate load groups based on their microscopy images (**Fig. 1A**). These shapes were isolated from the original tissue section by laser microdissectiona, and the proteome of 100 shapes equivalent to the volume of 10-15 complete hepatocytes was then acquired by mass spectrometry. We noted a 32-fold difference in AAT levels between low and high-load cells captured on the second principal component of a principle component analysis, following only the fibrosis stage represented on the first component (**Fig. 1B to 1D**). Given the sparsity of AAT+ cells in biopsy material, this validated the laser microdissection approach as it allowed the biological phenotype to emerge more clearly. Together with AAT, several known markers of AATD liver pathology were highly enriched in aggregate-positive cells, such as the 1.8-fold increased ER chaperone HSPA5 and the 2.8-fold increased ER-Golgi cargo receptor LMAN1 (**Fig. 1E**).

Pathway enrichment showed a strong elevation of proteins related to the three branches of unfolded protein response and a general upregulation of chaperones, with a concomitant reduction of the transcription and translation machinery (**Fig. 2A** **and** **2B**). Strikingly, many responses converged into a protective response to reactive oxygen species (ROS) with upregulation of thioredoxins, the ER-resident oxidoreductase ERO1A, and, importantly, and atypical increase in the peroxisomal compartment. The strong peroxisomal biogenesis response appeared early on, prominently featured by the peroxisomal proliferation factor PEX11B (**Fig. 2C** **and** **2D**). In contrast, most proteins of the core machinery of the unfolded protein response appeared only late during AAT build-up, despite visual protein accumulation at earlier stages (**Fig. 2E**). Of note, peroxisomal chaperones or chaperone-like proteins were not altered, making it unlikely that peroxisomes contribute to the clearance of unfolded proteins.

Samples were then stratified based on fibrosis status, finding that the peroxisomal response was almost exclusively present in all low-grade fibrosis (F1, n = 11) but virtually absent in high-grade fibrosis (F4, n = 6, **Fig. 2F**). This includes the proteins catalase (CAT) that detoxifies hydrogen peroxide, and PEX11B that induces peroxisomal gene expression and is interrelated to the signaling intensity of the Peroxisome proliferator-activated receptors (PPARs). Therefore, peroxisomal proliferation is a protective hallmark of hepatocytes in alpha-1 antitrypsin deficiency.

## Claims

1. A pharmaceutical composition comprising an agent for enhancing peroxisomes for use for prevention and/or treatment of a condition associated with and/or caused by alpha-1-antitrypsin deficiency (AATD).

2. The pharmaceutical composition of claim 1, **characterized in that** the agent for enhancing peroxisomes is a peroxisome proliferator-activated receptor (PPAR) agonist.

3. The pharmaceutical composition of claim 1 or claim 2, **characterized in that** the agent for enhancing peroxisomes is a PPAR-alpha agonist, a PPAR-gamma agonist, a PPAR-delta agonist and/or a PPAR-pan agonist.

4. The pharmaceutical composition of any one of claims 1 to 3 for use for prevention and/or treatment of a liver disease associated with and/or caused by AATD.

5. The pharmaceutical composition of any one of claims 1 to 4 for use for prevention and/or treatment of a proteotoxic and/or fibrogenic liver disease with AATD as a primary cause.

6. The pharmaceutical composition of any one of claims 1 to 5 for treatment or prevention of liver fibrosis with AATD as a primary cause.

7. The pharmaceutical composition of any one of claims 1 to 6, **characterized in that** the agent for enhancing peroxisomes is a PPAR-alpha agonist selected from 7(S)-hydroxydocosahexaenoic acid, bezafibrate, Clofibrate; Ciprofibrate; Fenofibrate; Fenofibric acid; Gemfibrozil; Oleoylethanolamide; Pemafibrate; 3-Hydroxy-2,2-dimethylbutyrate; 9-Hydroxy-10(E),12(E)-octadecadienoic acid; 9-Octadecenamide; Hexadecanamide; GW7647; LY-674; LY518674; and/or Wy-14643.

8. The pharmaceutical composition of any one of claims 1 to 6, **characterized in that** the agent for enhancing peroxisomes is a PPAR-pan agonist selected from Aleglitazar; Bezafibrate; Chiglitazar; Elafibranor; Lanifibranor; Lobeglitazone; Muraglitazar; Naveglitazar; Saroglitazar; Tesaglitazar; and/or ZLY18.

9. The pharmaceutical composition of any one of claims 1 to 6, **characterized in that** the agent for enhancing peroxisomes is a PPAR-delta agonist selected from GW0742; GW501516; KD3010; L165041; Seladelpar; Telmisartan; and/or 12 ((E)-2-(4-((3-(4-fluorophenyl)-3-(4-(3-morpholinoprop-1-yn-1-yl)phenyl)allyl)oxy)-2-methyl phenoxy) acetic acid).

10. The pharmaceutical composition of any one of claims 1 to 9, **characterized in that** the composition comprises the agent for enhancing peroxisomes in an amount of from 10mg to 800mg.

11. The pharmaceutical composition of any one of claims 1 to 9, **characterized in that** the agent for enhancing peroxisomes is administered in a daily amount of from 10mg to 1600mg.

12. An agent for enhancing peroxisomes, in particular a peroxisome proliferator-activated receptor (PPAR) agonist, for use for prevention and/or treatment of a disease associated with and/or caused by alpha-1-antitrypsin deficiency (AATD).

13. Use of an agent for enhancing peroxisomes, in particular of a peroxisome proliferator-activated receptor (PPAR) agonist, for the manufacture of a medicament for prevention and/or treating a disease associated with and/or caused by alpha-1-antitrypsin deficiency (AATD).

14. A method for prevention and/or treatment of a disease associated with and/or caused by alpha-1-antitrypsin deficiency (AATD) comprising administering an agent for enhancing peroxisomes, in particular a peroxisome proliferator-activated receptor (PPAR) agonist.
